# EUROPEAN PATENT APPLICATION

(11) **EP 4 094 714 A1**
(43) Date of publication of application: **30.11.2022**
(21) Application number: 21768638.5
(22) Date of filing: 18.02.2021
(51) Int. Cl.: A61B 90/20, G06T 1/00, G06T 5/00

(54) **MEDICAL IMAGE PROCESSING DEVICE AND MEDICAL OBSERVATION SYSTEM**

(30) Priority: 11.03.2020 JP 2020042474
(71) Applicant: Sony Olympus Medical Solutions Inc., Hachioji-shi, Tokyo 192-0904 (JP)
(72) Inventor: KADO, Masataka, Tokyo 192-0904 (JP)
(74) Representative: D Young & Co LLP
(86) International application number: PCT/JP2021/006220
(87) International publication number: WO 2021/182066

(57) **Abstract**

A medical image processing device of the present disclosure includes: a division unit configured to divide at least one subject image in an image; a detection unit configured to detect a blur of the subject image divided by the division unit; a correction unit configured to correct the blur of the subject image based on the subject image divided by the division unit and the blur detected by the detection unit; and a combining unit configured to combine the subject image after correction and a background image formed by a region other than the subject image.

## Description

### Field

The present disclosure relates to a medical image processing device and a medical observation system which perform image processing on image data input from the outside.

### Background

An optical microscope system including a support portion and a microscope portion (imaging unit) has been conventionally known as a medical observation system for observing a minute part in a brain, a heart, or the like of a patient, who is an object to be observed, at the time of performing an operation on the minute part. The support portion includes a plurality of arm portions. The microscope portion (imaging unit) is provided at a distal end of the support portion, and includes an enlarging optical system for enlarging the minute part and an imaging element. When performing an operation by using the microscope system, an operator (user) such as a doctor moves and arranges the microscope portion to a desired position, and performs an operation while observing an image captured by the microscope portion. Furthermore, an endoscope system including an endoscope unit (imaging unit) that images a surgical site is known as a medical observation system for observing the surgical site at the time of performing an operation in an abdominal cavity of a patient.

In contrast, a technique for correcting a blur of a subject in an image is known as a technique for easily viewing an image to be observed (e.g., see Patent Literature 1).

### Citation List

### Patent Literature

Patent Literature 1: JP 2014-17839 A

### Summary

### Technical Problem

Incidentally, an image captured by an imaging unit of a medical observation system has a plurality of subjects that independently move (vibrate), such as a surgical site and a treatment tool. Therefore, if blurs of the subjects in the image are uniformly corrected, the blurs may increase depending on how the subjects are blurred (blur direction and blur amount).

The present disclosure has been made in view of the above-described situation, and an object thereof is to provide a medical image processing device and a medical observation system capable of appropriately performing blur correction on an image having a plurality of subjects. Solution to Problem

To solve the above-described problem and achieve the object, a medical image processing device according to the present disclosure includes: a division unit configured to divide at least one subject image in an image; a detection unit configured to detect a blur of the subject image divided by the division unit; a correction unit configured to correct the blur of the subject image based on the subject image divided by the division unit and the blur detected by the detection unit; and a combining unit configured to combine the subject image after correction and a background image formed by a region other than the subject image.

Moreover, in the above-described medical image processing device according to the present disclosure, the division unit is configured to divide the subject image based on depth map information obtained by associating a distance between an imaging unit configured to capture an image and a subject with a position of the image.

Moreover, in the above-described medical image processing device according to the present disclosure, the combining unit is configured to: enlarge the subject image at a preset enlargement ratio; and combine the subject image after enlargement and the background image.

Moreover, in the above-described medical image processing device according to the present disclosure, the image includes a plurality of subject images, the division unit is configured to divide the plurality of subject images, the detection unit is configured to detect a blur of each of the plurality of subject images divided by the division unit, the correction unit is configured to correct the blur of each of the plurality of subject images based on each of the plurality of subject images divided by the division unit and the blur of each of the plurality of subject images detected by the detection unit, and the combining unit is configured to combine each of the plurality of subject images after correction and a background image formed by a region other than the plurality of subject images.

Moreover, a medical observation system according to the present disclosure includes: an imaging unit configured to image a surgical site of a patient on an operating table and output a video signal; a division unit configured to divide at least one subject image in an image generated based on the video signal; a detection unit configured to detect a blur of the subject image divided by the division unit; a correction unit configured to correct the blur of the subject image based on the subject image divided by the division unit and the blur detected by the detection unit; and a combining unit configured to combine the subject image after correction and a background image formed by a region other than the subject image. Advantageous Effects of Invention

According to the present invention, an effect of allowing appropriate blur correction on an image having a plurality of subjects is obtained.

### Brief Description of Drawings

FIG. 1 illustrates a configuration of a medical observation system according to an embodiment of the present disclosure.
FIG. 2 is a block diagram illustrating a configuration of a control device of the medical observation system according to the embodiment of the present disclosure.
FIG. 3 illustrates a use mode of a microscope device of the medical observation system according to the embodiment of the present disclosure.
FIG. 4 is a flowchart illustrating image processing performed by the control device of the medical observation system according to the embodiment of the present disclosure.
FIG. 5 illustrates division processing performed by a division unit (part 1).
FIG. 6 illustrates the division processing performed by the division unit (part 2).
FIG. 7 illustrates combining processing performed by the combining unit.
FIG. 8 illustrates a configuration of a medical observation system according to a variation of the present disclosure.

### Description of Embodiments

Embodiments for carrying out the present invention (hereinafter, referred to as "embodiments") will be described below with reference to the drawings. Note that the drawings are merely schematic, and portions having different dimensional relations and ratios may be included between the drawings.

### (Embodiment)

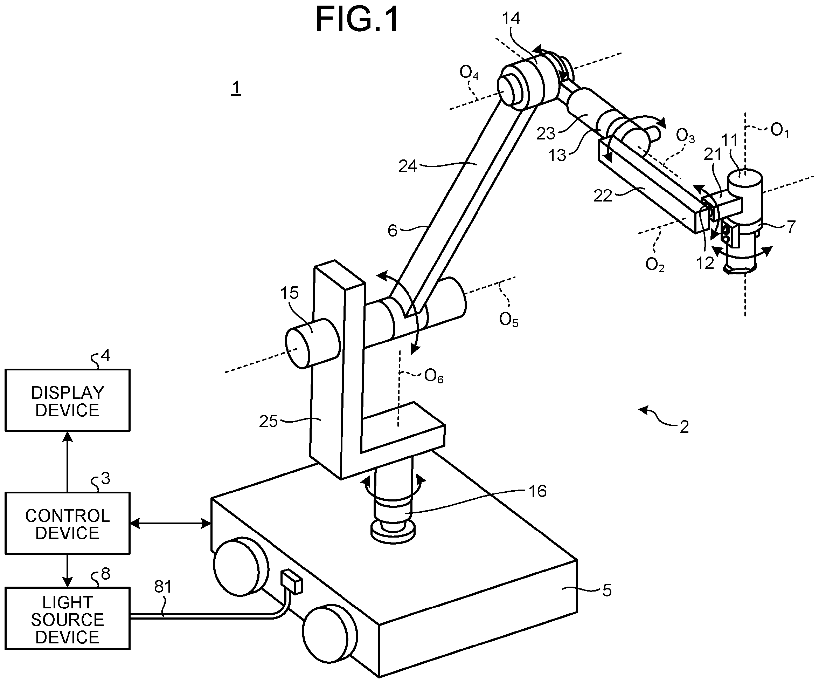

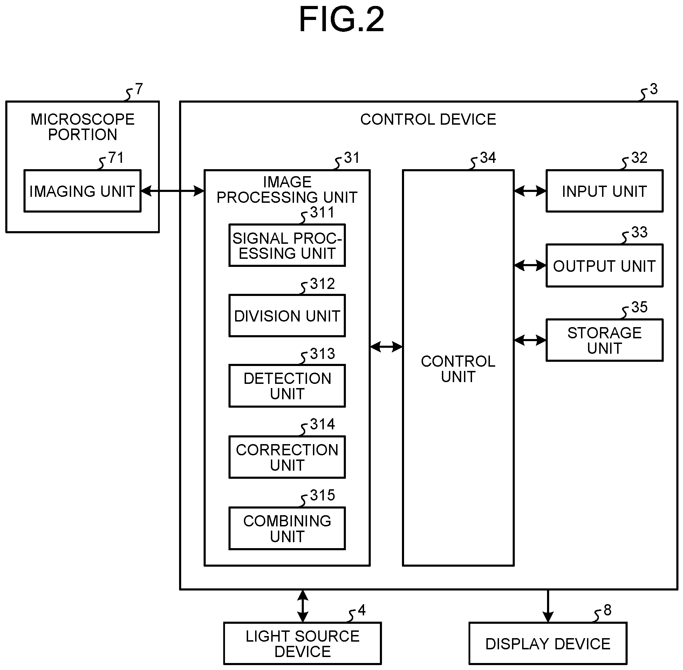

FIG. 1 illustrates a configuration of a medical observation system according to an embodiment. FIG. 2 is a block diagram illustrating a configuration of a control device of the medical observation system according to the embodiment. A medical observation system 1 includes a microscope device 2, a control device 3, a display device 4, and a light source device 8. The microscope device 2 has a function as a microscope that enlarges and images the microstructure of an object to be observed. The control device 3 integrally controls the operation of the medical observation system 1. The display device 4 displays an image captured by the microscope device 2. The light source device 8 supplies illumination light to the microscope device 2.

The microscope device 2 includes a base portion 5, a support portion 6, and a columnar microscope portion 7. The base portion 5 can move on a floor. The base portion 5 supports the support portion 6. The microscope portion 7 is provided at a distal end of the support portion 6, and enlarges and images a minute part of the object to be observed.

In the microscope device 2, for example, a cable group including a transmission cable, a light guide cable, and the like is disposed from the base portion 5 to the microscope portion 7. The transmission cable includes a signal line for transmitting a signal between the control device 3 and the microscope portion 7. The light guide cable guides illumination light from the light source device 8 to the microscope portion 7.

The support portion 6 includes a first joint portion 11, a first arm portion 21, a second joint portion 12, a second arm portion 22, a third joint portion 13, a third arm portion 23, a fourth joint portion 14, a fourth arm portion 24, a fifth joint portion 15, a fifth arm portion 25, and a sixth joint portion 16.

The support portion 6 includes four sets each including two arm portions and a joint portion that rotatably connects one (on distal end side) of the two arm portions to the other (on proximal end side). Specifically, these four sets includes (first arm portion 21, second joint portion 12, and second arm portion 22), (second arm portion 22, third joint portion 13, and third arm portion 23), (third arm portion 23, fourth joint portion 14, and fourth arm portion 24), and (fourth arm portion 24, fifth joint portion 15, and fifth arm portion 25) .

The first joint portion 11 rotatably holds the microscope portion 7 on the distal end side while being held by the first arm portion 21 on the proximal end side in a state of being fixed at a distal end portion of the first arm portion 21. The first joint portion 11 has a cylindrical shape, and rotatably holds the microscope portion 7 around a first axis O₁, which is a central axis in a height direction. The first arm portion 21 has a shape extending from a side surface of the first joint portion 11 in a direction orthogonal to the first axis O₁.

The second joint portion 12 rotatably holds the first arm portion 21 on the distal end side while being held by the second arm portion 22 on the proximal end side in a state of being fixed at a distal end portion of the second arm portion 22. The second joint portion 12 has a cylindrical shape, and rotatably holds the first arm portion 21 around a second axis O₂. The second axis O₂ is a central axis in a height direction, and orthogonal to the first axis O₁. The second arm portion 22 has a substantially L shape, and is connected to the second joint portion 12 at an end of a longitudinal portion of the L shape.

The third joint portion 13 rotatably holds a lateral portion of the L shape of the second arm portion 22 on the distal end side while being held by the third arm portion 23 on the proximal end side in a state of being fixed at a distal end portion of the third arm portion 23. The third joint portion 13 has a cylindrical shape, and rotatably holds the second arm portion 22 around a third axis O₃. The third axis O₃ is a central axis in a height direction, orthogonal to the second axis O₂, and parallel to a direction in which the second arm portion 22 extends. The third arm portion 23 has a cylindrical shape on the distal end side, and has a hole on the proximal end side. The hole penetrates in a direction orthogonal to a height direction of the cylinder on the distal end side. The third joint portion 13 is rotatably held by the fourth joint portion 14 via the hole.

The fourth joint portion 14 rotatably holds the third arm portion 23 on the distal end side while being held by the fourth arm portion 24 on the proximal end side in a state of being fixed to the fourth arm portion 24. The fourth joint portion 14 has a cylindrical shape, and rotatably holds the third arm portion 23 around a fourth axis O₄. The fourth axis O₄ is a central axis in a height direction, and orthogonal to the third axis O₃.

The fifth joint portion 15 rotatably holds the fourth arm portion 24 on the distal end side while being fixed and attached to the fifth arm portion 25 on the proximal end side. The fifth joint portion 15 has a cylindrical shape, and rotatably holds the fourth arm portion 24 around a fifth axis O₅. The fifth axis O₅ is a central axis in a height direction, and parallel to the fourth axis O₄. The fifth arm portion 25 includes an L-shaped portion and a rod-shaped portion extending downward from a lateral portion of the L shape. The fifth joint portion 15 is attached to an end of the longitudinal portion of the L shape of the fifth arm portion 25 on the proximal end side.

The sixth joint portion 16 rotatably holds the fifth arm portion 25 on the distal end side while being fixed and attached to the upper surface of the base portion 5 on the proximal end side. The sixth joint portion 16 has a cylindrical shape, and rotatably holds the fifth arm portion 25 around a sixth axis O₆. The sixth axis O₆ is a central axis in a height direction, and orthogonal to the fifth axis O₅. A proximal end portion of the rod-shaped portion of the fifth arm portion 25 is attached to the distal end side of the sixth joint portion 16.

The support portion 6 having the above-described configuration achieves movement of a total of six degrees of freedom of three degrees of freedom in translation and three degrees of freedom in rotation in the microscope portion 7.

Each of the first to six joint portions 11 to 16 has an electromagnetic brake that prohibits the rotation of the microscope portion 7 and the first to fifth arm portions 21 to 25. Each electromagnetic brake is released in a state where an arm operation switch (to be described later) provided in the microscope portion 7 is pressed, and the microscope portion 7 and the first to fifth arm portions 21 to 25 are permitted to rotate. Note that an air brake may be applied instead of the electromagnetic brake.

In addition to the above-described electromagnetic brake, an encoder and an actuator may be mounted in each joint portion. For example, when being provided in the first joint portion 11, the encoder detects a rotation angle around the first axis O₁. The actuator includes, for example, an electric motor such as a servomotor, is driven under the control of the control device 3, and causes rotation in the joint portion by a predetermined angle. The rotation angle in the joint portion is set by the control device 3 based on the rotation angle in each of rotation axes (first to sixth axes O₁ to O₆) as a value necessary for moving, for example, the microscope portion 7. As described above, the joint portion provided with an active driving system such as an actuator constitutes a rotation shaft that actively rotates by controlling the driving of the actuator.

The microscope portion 7 includes an imaging unit 71 in a cylindrical casing. The imaging unit 71 enlarges and captures an image of an object to be observed. In addition, the microscope portion 7 is provided with an arm operation switch and a cross lever. The arm operation switch receives operation input of releasing the electromagnetic brakes of the first to six joint portions 11 to 16 and permitting rotation of each joint portion. The cross lever can change magnification in the imaging unit and a focal length to the object to be observed. While a user presses the arm operation switch, the electromagnetic brakes of the first to six joint portions 11 to 16 are released.

The imaging unit 71 images a subject under the control of a camera head control unit 94. The imaging unit 71 houses a plurality of lenses and an imaging element in a casing. The imaging element receives a subject image formed by a lens, and converts the subject image into an electric signal (video signal). The imaging unit 71 forms an observation optical system. The observation optical system forms a subject image that has passed through the lens on an imaging surface of the imaging element.

In the embodiment, an image sensor and a TOF sensor is integrated to constitute the imaging element. The TOF sensor acquires subject distance information (hereinafter, referred to as depth map information) in a TOF method. The image sensor includes a charge coupled device (CCD) image sensor or a complementary metal oxide semiconductor (CMOS) image sensor. The depth map information is obtained by detecting, for each pixel position, a subject distance from the position of the imaging element to a corresponding position on an object to be observed corresponding to a pixel position in a captured image. Note that not only the TOF sensor but a phase difference sensor, a stereo camera, and the like may be adopted.

The light source device 8 controls emission of light under the control of the control device 3. The light source device 8 is connected to the microscope device 2 via a light source cable 81. An optical fiber is inserted into the light source cable 81.

The control device 3 receives an imaging signal output by the microscope device 2 and performs predetermined signal processing on the imaging signal to generate display image data. Note that the control device 3 may be installed inside the base portion 5, and integrated with the microscope device 2.

The control device 3 includes an image processing unit 31, an input unit 32, an output unit 33, a control unit 34, and a storage unit 35. Note that the control device 3 may be provided with, for example, a power supply unit (not illustrated) that generates a power supply voltage for driving the microscope device 2 and the control device 3, supplies the power supply voltage to each unit of the control device 3, and supplies the power supply voltage to the microscope device 2 via a transmission cable.

The image processing unit 31 performs processing on an imaging signal output by the microscope portion 7 to generate a display image. The image processing unit 31 includes a signal processing unit 311, a division unit 312, a detection unit 313, a correction unit 314, and a combining unit 315.

The signal processing unit 311 performs signal processing such as noise removal, A/D conversion, detection processing, interpolation processing, and color correction processing as necessary. The signal processing unit 311 generates an image signal before blur correction processing based on an imaging signal after the signal processing.

The division unit 312 extracts an image of a subject (subject image) appearing in the image before the blur correction generated by the signal processing unit 311, and divides a subject region including the extracted subject image. The image is divided into one or a plurality of subject regions and a background region obtained by excluding the subject regions by the division processing of the division unit 312. In the present embodiment, the division unit 312 extracts the subject image by using the depth map information. The division unit 312 divides a region surrounding the extracted subject as a subject region. The subject region may be set along the outer edge of the subject image, or may be set as a region moved outward by a predetermined distance from the outer edge of the subject image. Note that the subject may be extracted and divided by outline extraction by using an edge or image recognition processing by machine learning.

The detection unit 313 detects the motion of the subject in each subject region based on an image of a frame to be processed and an image that has been acquired before the image of the frame to be processed. The detection unit 313 detects a direction in which the subject moves and an amount of the motion for each subject region by, for example, frequency analysis. Furthermore, the detection unit 313 may detect the direction in which the subject moves and the amount of the motion by detecting a motion vector. A blur can be detected by a known method.

The correction unit 314 corrects a blur of the subject by performing the blur correction on the subject region. The correction unit 314 corrects the subject image based on the motion detected by the detection unit 313 for each subject region divided by the division unit 312. A blur can be corrected by a known method.

The combining unit 315 combines the subject region corrected by the correction unit 314 with the background region by superimposing an image of the subject region on the background region. For example, the combining unit 315 enlarges the corrected subject region at a preset enlargement ratio (> 1), and superimposes the enlarged subject region on a corresponding position of the image. The combined image generated by the combining unit 315 is output to the display device 4, and displayed on the display device 4.

Furthermore, the image processing unit 31 may include an AF processing unit and an AF arithmetic unit. The AF processing unit outputs a predetermined AF evaluation value of each frame based on an imaging signal of an input frame. The AF arithmetic unit performs AF arithmetic processing of selecting, for example, a frame or a focus lens position most suitable as a focusing position from the AF evaluation value of each frame from the AF processing unit.

The input unit 32 is implemented by using a user interface such as a keyboard, a mouse, and a touch panel, and receives inputs of various pieces of information.

The output unit 33 is implemented by using a speaker, a printer, a display, and the like, and outputs various pieces of information.

The control unit 34 controls the driving of each component including the control device 3 and a camera head 9, and controls input and output of information to each component, for example. The control unit 34 generates a control signal with reference to communication information data (e.g., communication format information) recorded in the storage unit 35, and transmits the generated control signal to the microscope device 2.

Note that the control unit 34 generates synchronization signals and clocks for the microscope portion 7 and the control device 3. A synchronization signal (e.g., synchronization signal that gives instruction on imaging timing) and a clock (e.g., clock for serial communication) for the microscope portion 7 are sent to the microscope portion 7 through a line (not illustrated). The microscope portion 7 is driven based on the synchronization signal and the clock.

The storage unit 35 is implemented by using a semiconductor memory such as a flash memory and a dynamic random access memory (DRAM), and stores communication information data (e.g., communication format information) and the like. Note that the storage unit 35 may record various programs and the like to be executed by the control unit 34, or may record depth map information acquired from the imaging unit 71 and a signal generated by the image sensor.

The image processing unit 31 and the control unit 34 described above are implemented by a general-purpose processor and a dedicated processor. The general-purpose processor includes, for example, a central processing unit (CPU) including an internal memory (not illustrated) in which a program is recorded. The dedicated processor includes, for example, various arithmetic circuits that execute a specific function, such as an application specific integrated circuit (ASIC). Furthermore, the image processing unit 31 and the control unit 34 may include a field programmable gate array (FPGA) (not illustrated), which is one type of programmable integrated circuit. Note that, when the image processing unit 31 and the control unit 34 include the FPGA, a memory for storing configuration data may be provided, and the FPGA, which is a programmable integrated circuit, may be configured by the configuration data read from the memory.

The display device 4 receives image data generated by the control device 3 from the control device 3, and displays an image corresponding to the image data. The above-described display device 4 includes a display panel including a cathode ray tube (CRT) display, liquid crystal, or organic electro luminescence (EL). Note that, an output device that outputs information by using a speaker, a printer, and the like may be provided in addition to the display device 4.

An operation performed by using the medical observation system 1 having the above-described configuration will be outlined. When an operator, who is a user, operates on a head of a patient, who is an object to be observed, the operator views an image displayed by the display device 4 while gripping the microscope portion 7 and moving the microscope portion 7 to a desired position with the arm operation switch of the microscope portion 7 being pressed to determine an imaging visual field of the microscope portion 7. Then, the operator releases his/her finger from the arm operation switch. This causes the electromagnetic brakes to be operated in the first to six joint portions 11 to 16. The imaging visual field of the microscope portion 7 is fixed. Then, the operator adjusts magnification and a focal length to the object to be observed, for example.

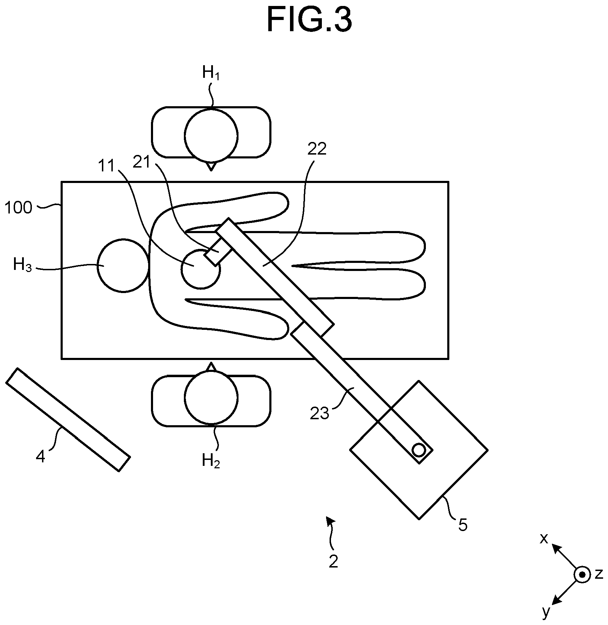

FIG. 3 illustrates a use mode of the microscope device of the medical observation system according to the embodiment of the present disclosure. Note that FIG. 3 illustrates a situation of an operation as viewed from directly above. An operator H₁ performs an operation while observing a video of a surgical site projected on the display device 4. The operator H₁ performs an operation on a patient H₃ lying on an operating table 100 by using the microscope device 2. Furthermore, in FIG. 3, an assistant H₂ who assists the operation is also illustrated in addition to the operator H₁ who performs the operation. Note that, in the present embodiment, an arrangement, in which the display device 4 is installed so as to be located substantially in front of the operator H₁ at the time of performing the operation in an upright position, is illustrated.

At the time of an operation, the operator H₁ treats the surgical site by using a treatment tool while the assistant H₂ may also assist by using a treatment tool. The microscope portion 7 captures an image including a treatment tool in an imaging region in addition to the surgical site.

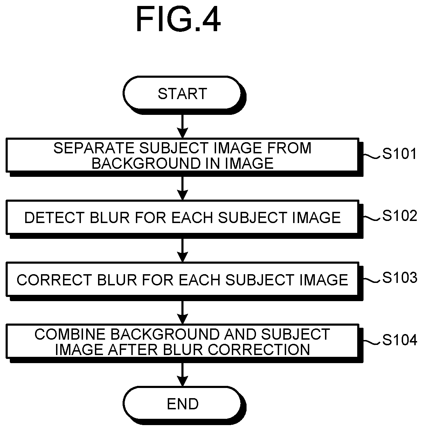

Next, blur correction processing performed by the image processing unit 31 will be described with reference to FIGS. 4 to 7. FIG. 4 is a flowchart illustrating image processing performed by the control device of the medical observation system according to the embodiment of the present disclosure. FIG. 4 illustrates a flow of performing blur correction on an image generated by the signal processing unit 311 and generating a display image.

First, in Step S101, the division unit 312 extracts a subject appearing in an image before blur correction generated by the signal processing unit 311, and divides a subject region including the extracted subject. For example, the division unit 312 extracts a subject image by using depth map information, and divides a subject image as the subject region.

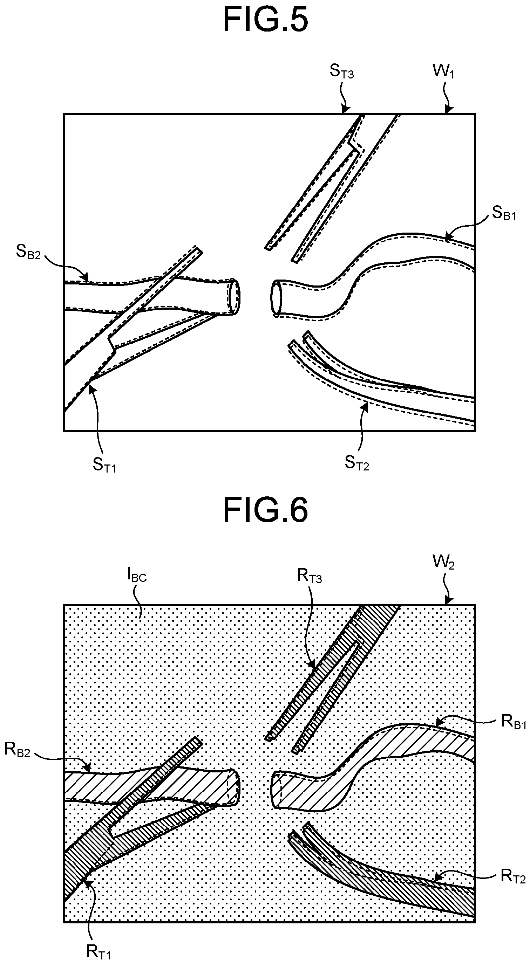

FIGS. 5 and 6 illustrate division processing performed by the division unit. In one example, a plurality of subjects wobbling in different motion amounts (vibration frequencies) and different movement directions appears in a pre-blur correction image W₁ in FIG. 5. Specifically, the pre-blur correction image W₁ indicates a blood vessel bypass operation. The pre-blur correction image W₁ includes images S_{B1} and S_{B2} of blood vessels (blood vessel images) to serve as a bypass, images S_{T1} and S_{T2} of treatment tools (treatment tool images) held by the operator, and an image S_{T3} of a treatment tool (treatment tool image) held by the assistant. The blood vessels and the treatment tools fluctuate in different amounts and directions. The fluctuation appears as a difference of a blur in each image. The division unit 312 divides the pre-blur correction image W₁ into a plurality of subject regions each including each image and a background region obtained by excluding each of the subject regions. Specifically, in the pre-blur correction image W₂ after the division, subject regions R_{B1}, R_{B2}, R_{T1}, R_{T2}, and R_{T3} are set. A region other than the subject regions R_{B1}, R_{B2}, R_{T1}, R_{T2}, and R_{T3} constitutes a background image I_{BC} (see FIG. 6). The region is obtained by excluding these subject regions. In FIG. 6, each subject region and the background image are hatched differently.

In Step S102, the detection unit 313 detects the motion of the subject in each subject region. The detection unit 313 detects a direction in which the subject moves and an amount of the motion for each subject region.

In Step S103, the correction unit 314 corrects a blur of the subject image by performing blur correction on the subject region. The correction unit 314 performs the correction based on the motion detected by the detection unit 313 for each subject region divided by the division unit 312.

In Step S104, the combining unit 315 combines the subject region corrected by the correction unit 314 with the background region by superimposing an image of the subject region on the background region. For example, the combining unit 315 enlarges the corrected subject region at a preset enlargement ratio (> 1), and superimposes the enlarged subject region on a corresponding position of the image.

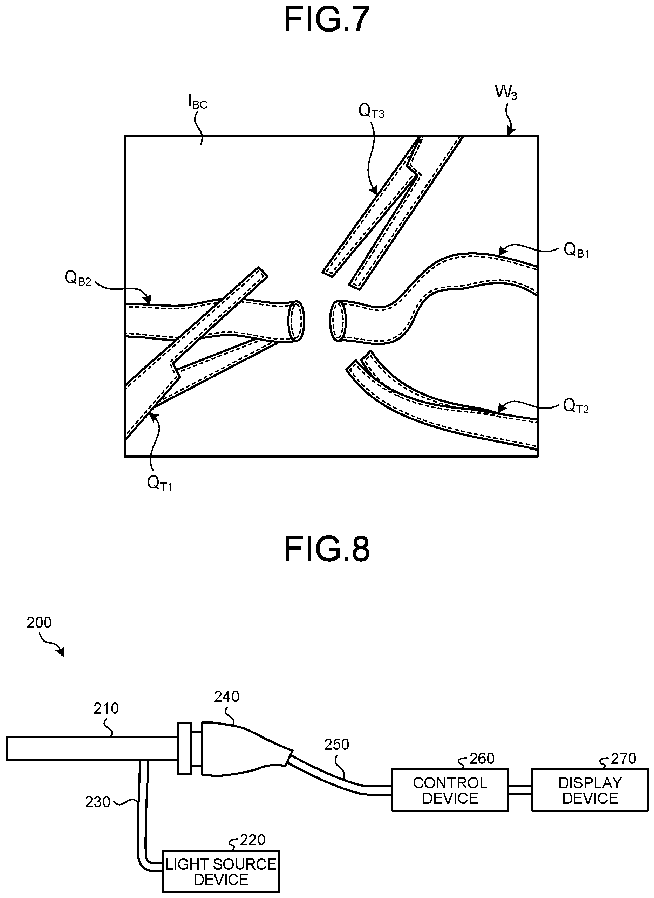

FIG. 7 illustrates combining processing performed by the combining unit. Note that broken lines in FIG. 7 indicate an outline of the subject image before enlargement. A combined image W₃ is generated by enlarging the blood vessel images (blood vessel images S_{B1} and S_{B2}) and the treatment tool images (treatment tool images S_{T1}, S_{T2}, and S_{T3}) after the blur correction and superimposing the blood vessel images (blood vessel images Q_{B1} and Q_{B2}) and the treatment tool images (treatment tool images Q_{T1}, Q_{T2}, and Q_{T3}), which have been enlarged, on the background image I_{BC}. When a subject image after the blur correction becomes partially smaller than a divided subject region and a gap is generated between the background and the subject, the gap can be filled by enlarging and superimposing the image of each subject.

In the above-described embodiment, in an image, images of subjects that vibrate (wobble) at different frequencies are divided, blur correction is individually performed, and then the subject images are combined to generate an image. Therefore, blur correction can be appropriately performed on an image having a plurality of subjects.

Note that, in the above-described embodiment, the signal processing unit 311 may uniformly perform the blur correction processing on the entire pre-blur correction image. In the blur correction processing, blurs of the entire image including a background are corrected, and blurs of the entire image due to vibrations of the microscope portion 7 and the operating table are corrected. This blur correction is performed before Step S101.

Furthermore, although, in the above-described embodiment, an example, in which the combining unit 315 enlarges a subject image after blur correction and superimposes the enlarged subject image on a background image, has been described, a background image may be interpolated based on values of surrounding pixels without enlarging the subject image to fill a gap between the subject image and the background, or the subject image (region) after the blur correction may be superimposed on the background image without enlarging the subject image.

Furthermore, although, in the above-described embodiment, an example, in which the combining unit 315 enlarges a subject image after blur correction at a preset enlargement ratio and superimposes the enlarged subject image on a background image, has been described, the enlargement ratio may be set in accordance with a blur amount, or the enlargement ratio may be set in accordance with a blur amount for each subject.

### (Variation)

Next, a variation of the present disclosure will be described with reference to FIG. 8. FIG. 8 illustrates a configuration of a medical observation system according to a variation. An endoscope device 200 will be described as the medical observation system according to the variation. The endoscope device 200 includes an insertion portion 210, a light source device 220, a light guide 230, a camera head 240, a cable 250, a control device 260, and a display device 270. In the variation, the insertion portion 210 and the camera head 240 correspond to a microscope portion. Furthermore, in the variation of the present disclosure, a support portion (support arm) that supports the endoscope device 200 may be provided.

The insertion portion 210 has an elongated shape, and internally includes an optical system that collects incident light. The distal end of the insertion portion 210 is inserted into a body cavity of a patient, for example. A rear end of the insertion portion 210 is detachably connected to the camera head 240. Furthermore, the insertion portion 210 is connected to the light source device 220 via the light guide 230. Light is supplied from the light source device 220.

In the variation, the depth map information is associated with optical information of the insertion portion 201, and obtained by detecting, for each pixel position, a subject distance from the position of the imaging element to a corresponding position on an object to be observed corresponding to a pixel position in a captured image. Here, unlike a microscope in which a viewpoint is fixed, a line-of-sight direction of observation during use (during operation) may change in the endoscope device 200. Therefore, when the depth map information is generated in the endoscope device 200, motion parallax and simultaneously localization and mapping (SLAM) may be used. Furthermore, the depth map information may be generated by controlling a support portion that supports the endoscope and imaging a surgical site from a plurality of viewpoints.

The light source device 220 is connected to the insertion portion 210 via the light guide 230. The light source device 220 supplies light to the insertion portion 210 via the light guide 230. Light supplied to the insertion portion 210 is emitted from the distal end of the insertion portion 210, and is applied to an object to be observed such as a tissue in the body cavity of a patient. Then, light reflected from the object to be observed is collected by the optical system in the insertion portion 210.

The camera head 240 has a configuration corresponding to the above-described imaging unit 71, and has a function of imaging the object to be observed. In the camera head 240, an imaging element, a lens, and the like are housed in a casing constituting the camera head 240. The camera head 240 is connected to the control device 260 via the cable 250. The camera head 240 images the object to be observed by photoelectrically converting light reflected from the object to be observed collected by the insertion portion 210, and outputs an image signal obtained by the imaging to the control device 260 via the cable 250.

The control device 260 controls the camera head 240, performs predetermined processing on the image signal output from the camera head 240, and then outputs the image signal to the display device 270. Similarly to the control device 3, the control device 260 includes the image processing unit 31, the input unit 32, the output unit 33, the control unit 34, and the storage unit 35. The control device 260 divides subject images that vibrate at different frequencies in the image, individually performs blur correction, and then combines the subject images to generate an image.

The display device 270 receives image data generated by the control device 260, and displays an image corresponding to the image data. The display device 270 includes, for example, a display panel including a CRT, liquid crystal, or organic EL.

According to the above-described variation, also in the endoscope device 200, in an image, images of subjects that vibrate (wobble) at different frequencies are divided, blur correction is individually performed, and then the subject images are combined to generate an image. Therefore, blur correction can be appropriately performed on an image having a plurality of subjects.

### (Other Embodiments)

Various inventions can be formed by appropriately combining a plurality of components disclosed in the medical observation system according to the above-described embodiment of the present disclosure. For example, some components may be deleted from all the components described in the medical observation system according to the above-described embodiment of the present disclosure. Moreover, the components described in the medical observation system according to the above-described embodiment of the present disclosure may be appropriately combined with each other.

Note that, although, in the description of the flowcharts in the present specification, the anteroposterior relation of processing between timings is clearly indicated by using expressions such as "first", "then", and "subsequently", the order of pieces of processing necessary for implementing the present disclosure is not uniquely determined by these expressions. That is, the order of pieces of processing in the flowcharts described in the present specification can be changed within a range without inconsistency.

Furthermore, in the medical observation system according to the embodiment of the present disclosure, the above-described "unit" can be replaced with a "device", a "circuit", and the like. For example, the control unit can be replaced with a control device or a control circuit.

Furthermore, a program to be executed by the medical observation system according to the embodiment of the present disclosure is provided by being recorded in a computer-readable recording medium such as a CD-ROM, a flexible disk (FD), a CD-R, a digital versatile disk (DVD), a USB medium, and a flash memory as file data in an installable format or an executable format.

Furthermore, the program to be executed by the medical observation system according to the embodiment of the present disclosure may be provided by being stored on a computer connected to a network such as the Internet and downloaded via the network.

Although some embodiments of the present application have been described in detail below with reference to the drawings, these embodiments are merely examples. The present invention can be implemented in other forms in which various modifications and improvements are made based on the knowledge of those skilled in the art, including the aspects described in the disclosure of the present invention.

Note that the present technology can also have the following configurations.
(1) A medical image processing device including:
   a division unit configured to divide at least one subject image in an image;
   a detection unit configured to detect a blur of the subject image divided by the division unit;
   a correction unit configured to correct the blur of the subject image based on the subject image divided by the division unit and the blur detected by the detection unit; and
   a combining unit configured to combine the subject image after correction and a background image formed by a region other than the subject image.
(2) The medical image processing device according to (1), wherein the division unit is configured to divide the subject image based on depth map information obtained by associating a distance between an imaging unit configured to capture an image and a subject with a position of the image.
(3) The medical image processing device according to (1) or (2), wherein the combining unit is configured to:
   enlarge the subject image at a preset enlargement ratio; and
   combine the subject image after enlargement and the background image.
(4) The medical image processing device according to any one of (1) to (3), wherein
   the image includes a plurality of subject images,
   the division unit is configured to divide the plurality of subject images,
   the detection unit is configured to detect a blur of each of the plurality of subject images divided by the division unit,
   the correction unit is configured to correct the blur of each of the plurality of subject images based on each of the plurality of subject images divided by the division unit and the blur of each of the plurality of subject images detected by the detection unit, and
   the combining unit is configured to combine each of the plurality of subject images after correction and a background image formed by a region other than the plurality of subject images.
(5) A medical observation system including:
   an imaging unit configured to image a surgical site of a patient on an operating table and output a video signal;
   a division unit configured to divide at least one subject image in an image generated based on the video signal;
   a detection unit configured to detect a blur of the subject image divided by the division unit;
   a correction unit configured to correct the blur of the subject image based on the subject image divided by the division unit and the blur detected by the detection unit; and
   a combining unit configured to combine the subject image after correction and a background image formed by a region other than the subject image.
(6) The medical observation system according to (5), wherein the division unit is configured to divide a subject image based on depth map information obtained by associating a distance between an imaging unit that captures an image and a subject with a position of the image.
(7) The medical observation system according to (5) or (6), wherein the combining unit is configured to:
   enlarge the subject image at a preset enlargement ratio; and
   combine the subject image after enlargement and the background image.
(8) The medical observation system according to any one of (5) to (7), wherein
   the image includes a plurality of subject images,
   the division unit is configured to divide the plurality of subject images,
   the detection unit is configured to detect a blur of each of the plurality of subject images divided by the division unit,
   the correction unit is configured to correct the blur of each of the plurality of subject images based on each of the plurality of subject images divided by the division unit and the blur of each of the plurality of subject images detected by the detection unit, and
   the combining unit is configured to combine each of the plurality of subject images after correction and a background image formed by a region other than the plurality of subject images.
(9) The medical observation system according to any one of (5) to (8), wherein
   the imaging unit is a microscope portion configured to image the surgical site of the patient, and
   the medical observation system further includes a support portion configured to support the microscope portion.
(10) The medical observation system according to any one of (5) to (8), wherein the imaging unit is an endoscope configured to image the surgical site of the patient.

### Industrial Applicability

As described above, a medical image processing device and the medical observation system according to the present invention are useful for appropriately performing blur correction on an image having a plurality of subjects. Reference Signs List

- 1: MEDICAL OBSERVATION SYSTEM
- 2: MICROSCOPE DEVICE
- 3, 260: CONTROL DEVICE
- 4,: 270 DISPLAY DEVICE
- 5: BASE PORTION
- 6: SUPPORT PORTION
- 7: MICROSCOPE PORTION
- 8,: 220 LIGHT SOURCE DEVICE
- 11: FIRST JOINT PORTION
- 12: SECOND JOINT PORTION
- 13: THIRD JOINT PORTION
- 14: FOURTH JOINT PORTION
- 15: FIFTH JOINT PORTION
- 16: SIXTH JOINT PORTION
- 21: FIRST ARM PORTION
- 22: SECOND ARM PORTION
- 23: THIRD ARM PORTION
- 24: FOURTH ARM PORTION
- 25: FIFTH ARM PORTION
- 31: IMAGE PROCESSING UNIT
- 32: INPUT UNIT
- 33: OUTPUT UNIT
- 34: CONTROL UNIT
- 35: STORAGE UNIT
- 71: IMAGING UNIT
- 81: LIGHT SOURCE CABLE
- 200: ENDOSCOPE DEVICE
- 210: INSERTION PORTION
- 230: LIGHT GUIDE
- 240: CAMERA HEAD
- 250: CABLE
- 311: SIGNAL PROCESSING UNIT
- 312: DIVISION UNIT
- 313: DETECTION UNIT
- 314: CORRECTION UNIT
- 315: COMBINING UNIT

## Claims

1. A medical image processing device comprising:
a division unit configured to divide at least one subject image in an image;
a detection unit configured to detect a blur of the subject image divided by the division unit;
a correction unit configured to correct the blur of the subject image based on the subject image divided by the division unit and the blur detected by the detection unit; and
a combining unit configured to combine the subject image after correction and a background image formed by a region other than the subject image.

2. The medical image processing device according to claim 1, wherein the division unit is configured to divide the subject image based on depth map information obtained by associating a distance between an imaging unit configured to capture an image and a subject with a position of the image.

3. The medical image processing device according to claim 1, wherein the combining unit is configured to:
enlarge the subject image at a preset enlargement ratio; and
combine the subject image after enlargement and the background image.

4. The medical image processing device according to claim 1, wherein
the image includes a plurality of subject images,
the division unit is configured to divide the plurality of subject images,
the detection unit is configured to detect a blur of each of the plurality of subject images divided by the division unit,
the correction unit is configured to correct the blur of each of the plurality of subject images based on each of the plurality of subject images divided by the division unit and the blur of each of the plurality of subject images detected by the detection unit, and
the combining unit is configured to combine each of the plurality of subject images after correction and a background image formed by a region other than the plurality of subject images.

5. A medical observation system comprising:
an imaging unit configured to image a surgical site of a patient on an operating table and output a video signal;
a division unit configured to divide at least one subject image in an image generated based on the video signal;
a detection unit configured to detect a blur of the subject image divided by the division unit;
a correction unit configured to correct the blur of the subject image based on the subject image divided by the division unit and the blur detected by the detection unit; and
a combining unit configured to combine the subject image after correction and a background image formed by a region other than the subject image.

6. The medical observation system according to claim 5, wherein the division unit is configured to divide a subject image based on depth map information obtained by associating a distance between an imaging unit that captures an image and a subject with a position of the image.

7. The medical observation system according to claim 5, wherein the combining unit is configured to:
enlarge the subject image at a preset enlargement ratio; and
combine the subject image after enlargement and the background image.

8. The medical observation system according to claim 5, wherein
the image includes a plurality of subject images,
the division unit is configured to divide the plurality of subject images,
the detection unit is configured to detect a blur of each of the plurality of subject images divided by the division unit,
the correction unit is configured to correct the blur of each of the plurality of subject images based on each of the plurality of subject images divided by the division unit and the blur of each of the plurality of subject images detected by the detection unit, and
the combining unit is configured to combine each of the plurality of subject images after correction and a background image formed by a region other than the plurality of subject images.

9. The medical observation system according to claim 5, wherein
the imaging unit is a microscope portion configured to image the surgical site of the patient, and
the medical observation system further comprises a support portion configured to support the microscope portion.

10. The medical observation system according to claim 5, wherein the imaging unit is an endoscope configured to image the surgical site of the patient.
